# EUROPEAN PATENT APPLICATION

(11) **EP 4 029 504 A1**
(43) Date of publication of application: **20.07.2022**
(21) Application number: 21867879.5
(22) Date of filing: 19.10.2021
(51) Int. Cl.: A61K 31/635, A61K 45/06, A61P 17/00, A61P 17/06, A61P 17/18, A61P 17/04, A61P 17/02, A61P 35/00

(54) **USE OF BCL-2 INHIBITOR AND PHARMACEUTICAL COMPOSITION FOR TREATING AGING-RELATED SKIN DISEASE**

(30) Priority: 19.11.2020 CN 202011301947; 20.01.2021 CN 202110074555
(71) Applicant: XEON BIOPHARMACEUTICAL LIMITED, Suzhou, Jiangsu 215000 (CN); NANCHANG ANTI-SENESCENCE BIOLOGICAL & MEDICAL COMPANY, Nanchang, Jiangxi 330000 (CN)
(72) Inventor: LU, Qianjin, Hunan 410011 (CN); WU, Haijing, Hunan 410011 (CN); ZHU, Huan, Hunan 410011 (CN); JIANG, Jiao, Hunan 410011 (CN); ZHAO, Ming, Hunan 410011 (CN); LI, Wenqun, Hunan 410011 (CN)
(74) Representative: Kador & Partner PartG mbB
(86) International application number: PCT/CN2021/124587
(87) International publication number: WO 2022/105511

(57) **Abstract**

The present disclosure discloses use of a B-cell lymphoma 2 (BCL-2) inhibitor for treating aging-related skin diseases and a pharmaceutical composition for treating the aging-related skin diseases, belonging to the technical field of medicine. In the present disclosure, studies have found that pharmaceutical compositions containing the BCL-2 inhibitor can significantly ameliorate skin erythema and scale in imiquimod-induced psoriasis mouse models, significantly reduce a thickness of skin lesions, and decrease expression of aging-related proteins P16 and P21 and mRNA expression levels of *p16*, *p21* and other genes in the skin lesions of the psoriasis mouse models. In addition, the pharmaceutical composition containing the BCL-2 inhibitor can significantly reduce infiltration of CD4⁺ T cells in dermis of the skin lesions of the psoriasis mouse models, and mRNA expression levels of skin lesion inflammatory factors such as IFN-γ, IL-17A, IL-22 and IL-23. Moreover, the pharmaceutical composition is superior to traditional intraperitoneal injection methods. It can be seen that the BCL-2 inhibitor and the pharmaceutical composition can effectively ameliorate the psoriasis mouse models, and have a prospect of developing into drugs for treating the psoriasis and the aging-related skin diseases.

## Description

### CROSS REFERENCE TO RELATED APPLICATION(S)

This application claims priority to Chinese Patent Application No. 202011301947.8, filed with the China National Intellectual Property Administration on Thursday, November 19, 2020 and entitled "USE OF B-CELL LYMPHOMA 2 (BCL-2) INHIBITOR AND PHARMACEUTICAL COMPOSITION FOR TREATING AGING-RELATED SKIN DISEASES", which is herein incorporated by reference in its entirety.

### TECHNICAL FIELD

The present disclosure belongs to the technical field of medicine, and specifically relates to use of a BCL-2 inhibitor and a pharmaceutical composition for treating aging-related skin diseases.

### BACKGROUND ART

Cellular senescence refers to that cell proliferation and differentiation and cellular physiological function gradually decline during life activities over time. Senescent cells undergo morphological and metabolic changes, chromatin remodeling, gene expression changes, cell cycle arrest and secretion of senescence-associated secretory phenotypes (SASPs). The senescent cells are widely present in aging people and disease tissues, and studies have found that the senescent cells may be pivotal in occurrence and progression of many diseases, such as cancer, Alzheimer's disease, Parkinson's disease, atherosclerosis, cardiovascular dysfunction and inflammatory diseases.

In recent years, the senescent cells have received much attention in pathogenesis of the diseases. Aging-related skin diseases include eczema, psoriasis, hyperpigmentation, mole, rash, atopic dermatitis, urticaria, diseases and disorders related to photosensitivity or photoaging and wrinkles, itching, dullness, eczema outbreak, eosinophilic skin disease, reactive neutrophil skin disease, pemphigus; pemphigoid, immune bullous skin disease, skin fibrous tissue cell proliferation, keloids, skin lymphoma and skin diseases or conditions of cutaneous lupus. The aging-related skin diseases will be described in detail with the psoriasis as an example.

Psoriasis is a chronic inflammatory skin disease with abnormal proliferation of keratinocytes. The psoriasis has a very complex pathogenesis, which is considered as a result of a combination of genetic, epigenetic and environmental factors. Psoriasis vulgaris is the most common type of the psoriasis, accounting for nearly 85-90% of all psoriasis cases. Histopathologically, psoriasis is characterized by excessive proliferation and abnormal differentiation of the keratinocytes, new capillary dilation and inflammatory infiltration of dermis. In terms of clinical features, psoriasis is manifested as recurring multilayer silver-white dry scales, with extensive infiltrating erythema and bleeding points under the scales, which may be accompanied by itching symptoms. The psoriasis has a high incidence, and China has a total prevalence of about 0.59% [referring to Epidemiological Survey of Psoriasis in Six Provinces and Cities in China, Xiaolan Ding]. Recent studies have found that senescence-related proteins P16 and senescence-associated secretory phenotypes (SASPs) are up-regulated in the skin of patients with psoriasis. This is one of the important evidences that senescent cells play a role in pathogenesis of the psoriasis.

At present, the psoriasis is mainly treated by traditional therapies and biological targeted therapies according to the types of drugs. Mostly, the traditional therapies can only reach a short-term clinical effect and is difficult to cure the diseases, while most of the therapies are accompanied by adverse reactions of varying degrees. The biological targeted therapies have higher efficacy and better safety than the traditional therapies, but some patients still have poor therapeutic effects or adverse reactions; in addition, some biological preparations are expensive. From the way of medication, there are mainly manners of topical administration and systemic administration. Some of topical administration methods are effective in controlling psoriasis-caused skin lesions; and from the perspective of safety, the topical administration is better than systemic treatments. For treating the patients with psoriasis, it is of great significance to develop safe, efficient and economical novel drugs for the psoriasis.

### SUMMARY

A first objective of the present disclosure is to provide novel use of a BCL-2 inhibitor in treatment of aging-related skin diseases, especially psoriasis.

A second objective of the present disclosure is to provide a drug containing the BCL-2 inhibitor for treatment of aging-related skin diseases; and in particular, the present disclosure relates to a local injection or an external preparation containing the BCL-2 inhibitor for treatment of psoriasis.

The present disclosure adopts the technical solutions as follows.

Use of a BCL-2 inhibitor in preparation of a drug for treating aging-related skin diseases is provided.

The present disclosure provides the novel use of the BCL-2 inhibitor in the treatment of the aging-related skin diseases.

In the present invention, the BCL-2 inhibitor can inhibit members of a BCL-2 anti-apoptotic protein family.

Preferably, the members of a BCL-2 anti-apoptotic protein family may include at least one selected from the group consisting of BCL-2, BCL-xL and BCL-w.

Preferably, the BCL-2 inhibitor may be at least one selected from the group consisting of ABT-737 and ABT-263, further preferably ABT-737.

In the present invention, any pharmaceutically-acceptable pharmaceutical preparation is prepared by combining a pharmaceutically-effective amount of the BCL-2 inhibitor with pharmaceutically-acceptable accessories.

In the present invention, an inhibitor including one or more members of a BCL-2 anti-apoptotic protein family (such as BCL-2, BCL-xL and/or BCL-w) is combined with all pharmaceutically-acceptable carriers or excipients, to prepare any pharmaceutically-acceptable pharmaceutical preparation based on existing preparation equipment and methods.

Preferably, the any pharmaceutical preparation may be prepared by the BCL-2 inhibitor for external, topical, transdermal, intradermal, systemic, inhalation, intratracheal or intubation administrations.

Preferably, the pharmaceutical preparation may be at least one selected from the group consisting of an injection preparation and an external preparation.

Preferably, the injection preparation may be a subcutaneous injection preparation.

Preferably, the external preparation may be at least one selected from the group consisting of gels, emulsifiable pastes, emulsions, creams, ointments, pastes, lotions, patches, dressings, coatings, cataplasms, aerosols and sprays.

Preferably, the BCL-2 inhibitor may be used in preparation of a drug for aging-related skin diseases in progressive, stationary or regressive phases.

In the present invention, the aging-related skin diseases include at least one selected from the group consisting of eczema, psoriasis, hyperpigmentation, mole, rash, atopic dermatitis, urticaria, diseases and disorders related to photosensitivity or photoaging and wrinkles, itching, dullness, eczema outbreak, eosinophilic skin disease, reactive neutrophil skin disease, pemphigus; pemphigoid, immune bullous skin disease, skin fibrous tissue cell proliferation, keloids, skin lymphoma and cutaneous lupus.

Preferably, the aging-related skin disease may be psoriasis.

Further preferably, the psoriasis may be at least one selected from the group consisting of psoriasis vulgaris, erythroderma psoriasis, psoriatic arthritis and pustular psoriasis.

Preferably, the BCL-2 inhibitor may be combined with other drugs for treating the aging-related skin diseases to prepare a composite drug.

The present disclosure further provides a drug for treating aging-related skin diseases, including a pharmaceutically-effective amount of a BCL-2 inhibitor.

In the present disclosure, the drug for treating aging-related skin diseases includes one or more combined active ingredients of the BCL-2 inhibitor.

In the present disclosure, the BCL-2 inhibitor can inhibit members of a BCL-2 anti-apoptotic protein family. Preferably, the members of a BCL-2 anti-apoptotic protein family may include at least one selected from the group consisting of BCL-2, BCL-xL and BCL-w; preferably, the BCL-2 inhibitor may be at least one selected from the group consisting of ABT-737 and ABT-263, further preferably ABT-737.

In the present disclosure, the drug for treating aging-related skin diseases is any pharmaceutically-acceptable pharmaceutical preparation. For example, the any pharmaceutical preparation is prepared for external, topical, transdermal, intradermal, systemic, inhalation, intratracheal or intubation administrations.

Preferably, the drug may have a dosage form of an injection preparation or an external preparation;
preferably, the injection preparation may be a subcutaneous injection preparation; and
preferably, the external preparation may be at least one selected from the group consisting of gels, emulsifiable pastes, emulsions, creams, ointments, pastes, lotions, patches, dressings, coatings, cataplasms, aerosols or sprays.

In the present disclosure, the injection preparation includes the BCL-2 inhibitor, a solvent A, a solvent B, a solvent C and a surfactant;
preferably, the solvent A may be at least one organic solvent selected from the group consisting of dimethyl sulfoxide (DMSO), dimethyl formamide (DMF), dimethyl acetamide (DMAC or DMA), N-methylpyrrolidone (NMP) and DMAF;
preferably, the solvent B may be one or more alcohol solvents selected from the group consisting of PEG-300, PEG-400, polyethylene glycol or propylene glycol, ethanol and glycerin;
preferably, the solvent C may be water or a buffer, preferably at least one selected from the group consisting of a phosphate-buffered saline (PBS), a carbonate buffer, an acetate buffer, a borate buffer, a citrate buffer, a citrate buffer and purified water;
preferably, the surfactant may be at least one selected from the group consisting of polysorbate, Tween-80, Tween-20, polyoxy castor oil, nonionic surfactant HS15, vitamin E polyethylene glycol succinate, Pluronic F68 and gelucire 44/14;
preferably, in the injection preparation, the BCL-2 inhibitor may have a mass percentage of 0.001-5%, preferably 0.05-1%;
the solvent A may have a mass percentage of 0.1-15%, preferably 5-10%;
the solvent B may have a mass percentage of 20-60%;
the surfactant may have a mass percentage of 1-10%; and
the solvent C may be used as a balance;
preferably, optionally the external preparation may further include other accessories that are allowed to be added in the injection preparation, for example, at least one of a penetration enhancer and a preservative.

Preferably, the penetration enhancer may be one or more selected from the group consisting of menthol, borneol, azone, oleic acid, and *Cnidium monnieri* volatile oil; and the penetration enhancer may have a weight percentage of not more than 10%; and
preferably, the preservative may be at least one selected from the group consisting of ethyl paraben, ethyl paraben, sodium benzoate, benzalkonium bromide, chlorobutanol and sorbic acid; and the preservative may have a weight percentage of not more than 10%.

The present disclosure has further developed an external preparation, and innovatively discovered that the BCL-2 inhibitor for external application is helpful to new mechanisms: delaying disease progress by down-regulating BCL-2 and TET-2 to clear senescent CD4⁺ T lymphocytes; and reducing mRNA levels of inflammatory factors, such as Il 17a, Il 17f, 1122 and 1123, thereby alleviating the diseases to further improve therapeutic effects of the aging-related skin diseases, especially the psoriasis. In addition, the research of the present disclosure found that the external preparation has therapeutic effects in improving the aging-related skin diseases, especially the psoriasis, significantly better than the traditional systemic administration methods by intraperitoneal injection, and helps to reduce the inevitable toxic and side effects of other administration routes.

The external preparation includes the BCL-2 inhibitor, a solvent D, a substrate and a solvent E;
the solvent D is one or more selected from the group consisting of DMSO, DMF, DMAC or DMA, NMP and DMAF;
the substrate is one or more selected from the group consisting of hydroxypropyl methylcellulose, sodium carboxymethyl cellulose, carbomer, hyaluronic acid, polyvinyl alcohol, sodium alginate, sodium hyaluronate, methylcellulose, chitosan and chitin; and
the solvent E is one or more selected from the group consisting of a PBS, a carbonate buffer, an acetate buffer, a borate buffer, a citrate buffer, a citrate buffer and purified water.

Preferably, in the external preparation, the BCL-2 inhibitor may have a weight percentage of 0.001-5%, preferably 0.02-1%;
the solvent D may have a weight percentage of 0.1-15%, preferably 0.5-1.5%;
the substrate may have a weight percentage of 0.01-10%, preferably 0.1-1.0%; and
the solvent E may be used as a balance;
preferably, optionally the external preparation may further include other accessories that are allowed to be added in the external preparation, for example, at least one of a humectant, a solvent F, a penetration enhancer and a preservative;
preferably, the humectant may be selected from the group consisting of propylene glycol and glycerin; the humectant may have a weight percentage of not more than 10%; and
preferably, the solvent F may be one or more selected from the group consisting of PEG-300, PEG-400, polyethylene glycol, propylene glycol, ethanol and glycerin; preferably, the solvent F may have a weight percentage of not more than 10%;
preferably, the penetration enhancer may be one or more selected from the group consisting of menthol, borneol, azone, oleic acid, and *Cnidium monnieri* volatile oil; and the penetration enhancer may have a weight percentage of not more than 10%; and
preferably, the preservative may be at least one selected from the group consisting of ethyl paraben, ethyl paraben, sodium benzoate, benzalkonium bromide, chlorobutanol and sorbic acid; and the preservative may have a weight percentage of not more than 10%.

In the present disclosure, the drug further includes other active ingredients for treating the aging-related skin diseases. The BCL-2 inhibitor and the pharmaceutical composition can be used for a monotherapy or a combination therapy. The BCL-2 inhibitor and other ingredients can be combined to further improve the therapeutic effects of aging-related skin diseases.

It should be noted that within the technical scope disclosed by the present disclosure, the addition or deletion of auxiliary medicinal ingredients or the changes of dosage form, the changes or deletion of proportion of each component, the addition of excipients to the pharmaceutical composition, or the equivalent replacements or changes according to the technical solution of the present disclosure and its inventive concept, shall be all covered within the protection scope of the present disclosure.

In the present disclosure, the injections and external preparations of the BCL-2 inhibitor can be locally absorbed to remove senescent cells from lesions of aging-related skin diseases and control development of the diseases. Meanwhile, in terms of safety, the injections and external preparations of the BCL-2 inhibitor are safer than systemic drugs.

In the present disclosure, the drug can show desirable therapeutic effects against aging-related skin diseases in the progressive, stationary and regressive phases.

In the present disclosure, the drug and the pharmaceutical composition alleviate the skin erythema, scales and other symptoms of the psoriasis mouse models induced by imiquimod and reduce the thickness of the epidermis; the drug and the pharmaceutical composition reduce the expression of aging-related proteins P16 and P21 and the mRNA expression levels of genes such as *p16* and *p21* in the skin lesions of the psoriasis mouse models, to improve the skin aging of the psoriasis mouse models; the drug and the pharmaceutical composition can significantly reduce infiltration of CD4⁺ T cells in dermis of the skin lesions of the psoriasis mouse models, and the mRNA expression levels of skin lesion inflammatory factors such as IFN-γ, IL-17A, IL-22 and IL-23. In addition, the pharmaceutical composition has therapeutic effects in improving the aging-related skin diseases, especially the psoriasis, significantly better than that of the traditional systemic administration methods by intraperitoneal injection. Moreover, it is innovatively discovered that the drug and the pharmaceutical composition significantly reduce the mRNA expression levels of Tet2 in skin tissues of psoriasis mice.

In the above-mentioned medical uses, an administration time, a number of administrations and an administration frequency of the BCL-2 inhibitor and the pharmaceutical composition are determined according to specific diagnosis results of the diseases. This is within the technical scope of those skilled in the art.

The treatment plan for mice is applied to the human body, and an effective dose of all drugs to humans can be converted by the effective dose of the drug to mice. This is also easy to achieve for those of ordinary skill in the art.

In order to better understand the essence of the present disclosure, new uses of the BCL-2 inhibitor and the pharmaceutical composition in the pharmaceutical field are further illustrated using animal experiment results in the following examples.

The present disclosure has the following advantages over the prior art:

In the present disclosure, the BCL-2 inhibitor and two pharmaceutical compositions containing the BCL-2 inhibitor are used for treating aging-related skin diseases, with low cost and convenient use; meanwhile, the pharmaceutical composition has a therapeutic effect in improving the aging-related skin diseases, especially the psoriasis, significantly better than that of the traditional systemic administration methods by intraperitoneal injection; in addition, the injection or the external administration avoids the adverse effects of systemic treatment; taking psoriasis as an example, the test results show that the drug can significantly ameliorate skin inflammatory responses of mouse models with imiquimod-induced psoriasis, relieve symptoms such as skin erythema and scales, and significantly reduce the thickness of skin lesions. In addition, the two BCL-2-based pharmaceutical compositions can significantly reduce the expression of aging-related proteins P16 and P21 in the skin lesions of psoriasis mice, as well as the mRNA expression levels of genes such as *p16* and *p21*, to improve the skin aging in psoriasis mouse models; the two BCL-2-based pharmaceutical compositions can significantly reduce infiltration of CD4⁺ T cells in dermis of the skin lesions of the psoriasis mouse models, and the mRNA expression levels of skin lesion inflammatory factors such as IFN-γ, IL-17A, IL-22 and IL-23; meanwhile, compared with the traditional intraperitoneal administration methods, the pharmaceutical composition significantly improves the disease phenotype of psoriasis, the pathological staining of HE, the expression of senescence-related proteins P16 and P21, and mRNA levels of genes such as *p16*, *p21* and various inflammatory factors. In addition, the drug and the pharmaceutical composition significantly reduce the mRNA expression levels of Tet2 in the skin tissues of mice with psoriasis.

In the present disclosure, studies have found that for psoriasis, the external administration of the BCL-2 inhibitor helps to show a better therapeutic effect based on a new mechanism, is better than the traditional intraperitoneal administration methods, and helps to further reduce side effects.

### BRIEF DESCRIPTION OF THE DRAWINGS

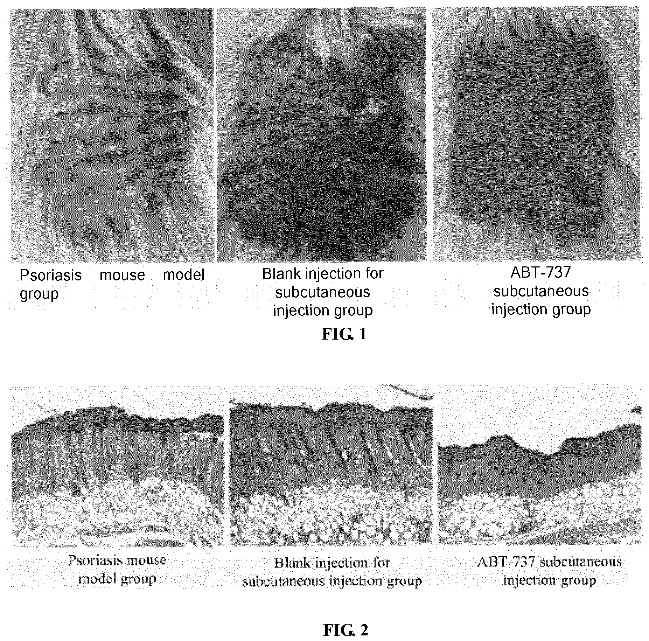

FIG. 1 is a picture of skin lesions on a back of a mouse in a psoriasis mouse model group, a blank injection for subcutaneous injection group, and an ABT-737 subcutaneous injection group provided in Example 5 of the present disclosure on a 7th day of imiquimod modeling;
FIG. 2 is an HE image of skin lesion sections of a mouse back modeling in the psoriasis mouse model group, the blank injection for subcutaneous injection group, and the ABT-737 subcutaneous injection group provided in Example 5 of the present disclosure;

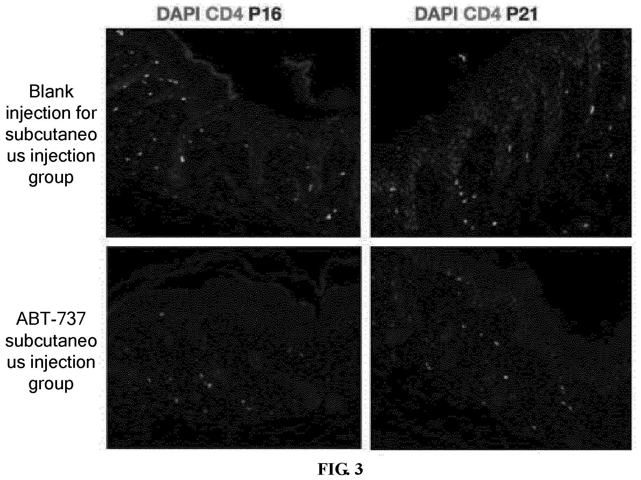

Figure 3 is an immunohistochemical staining map of aging-related proteins P16 and P21 and CD4 molecules in paraffin sections of skin lesions on a back of a mouse back modeling in the blank injection for subcutaneous injection group and the ABT-737 subcutaneous injection group provided in Example 5 of the present disclosure;

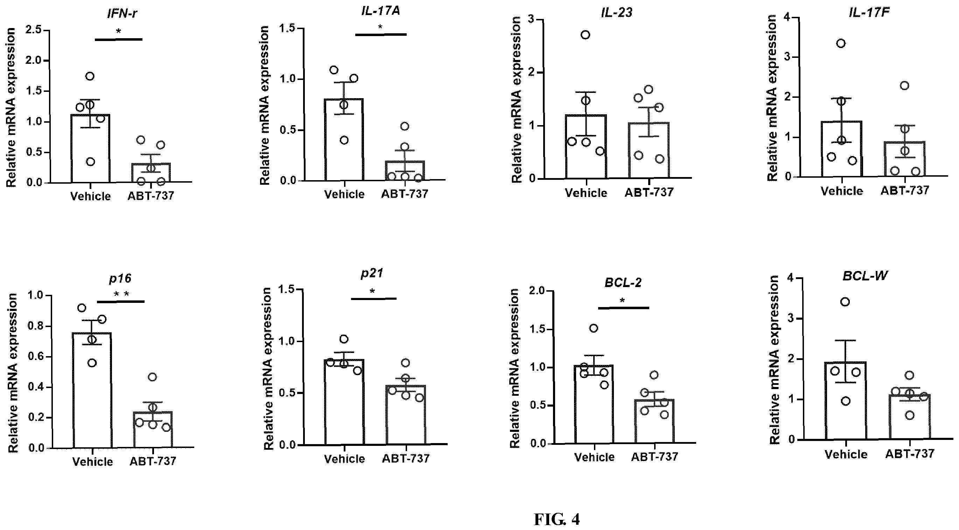

FIG. 4 is an mRNA expression level map of aging-related molecules P16 and P21, BCL-2 and each inflammatory factor gene of skin lesions on a mouse back modeling in the blank injection for subcutaneous injection group and the ABT-737 subcutaneous injection group provided in Example 5 of the present disclosure by a real-time quantitative PCR technology;

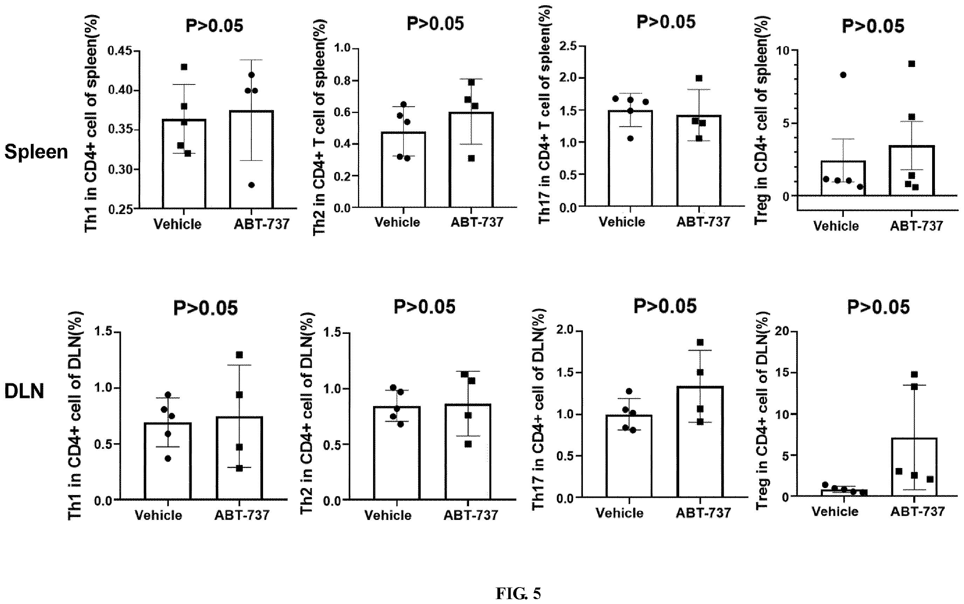

FIG. 5 is a diagram showing proportions of Th1, Th2, Th17 and Treg cells in mouse spleen and draining lymph nodes (DLNs) in the blank injection for subcutaneous injection group and the ABT-737 subcutaneous injection group provided in Example 5 of the present disclosure by flow cytometry;

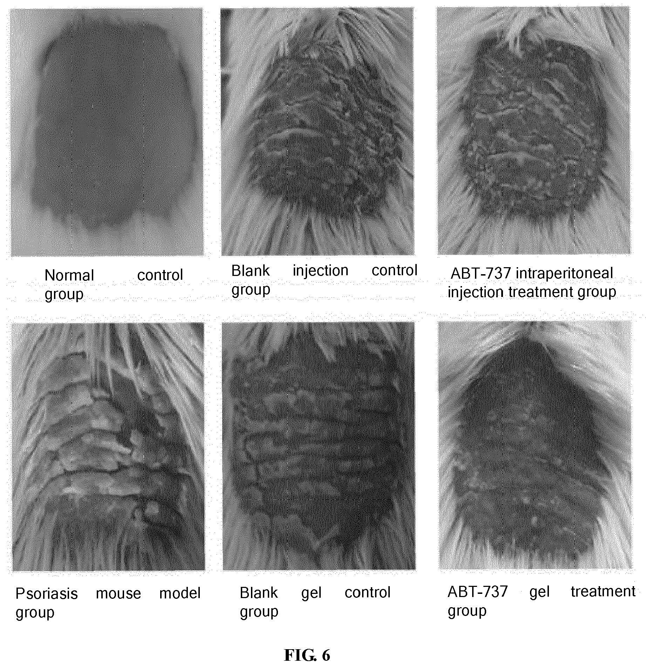

FIG. 6 is a picture of skin lesions on a mouse back modeling on a 7th day of a psoriasis mouse model group, a blank injection for intraperitoneal injection group, an ABT-737 intraperitoneal injection treatment group, a blank gel control group, and an ABT-737 gel treatment group provided in Example 6 of the present disclosure;

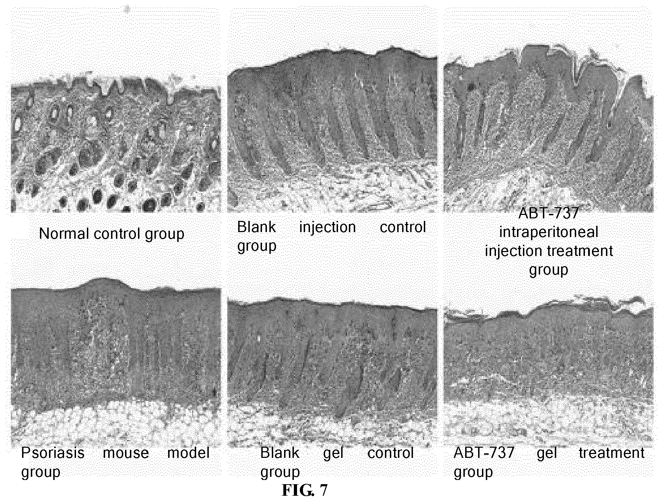

FIG. 7 is an HE image of skin lesion sections at a mouse back modeling on a 7th day in the psoriasis mouse model group, the blank injection for intraperitoneal injection group, the ABT-737 intraperitoneal injection treatment group, the blank gel control group, and the ABT-737 gel treatment group provided in Example 6 of the present disclosure;

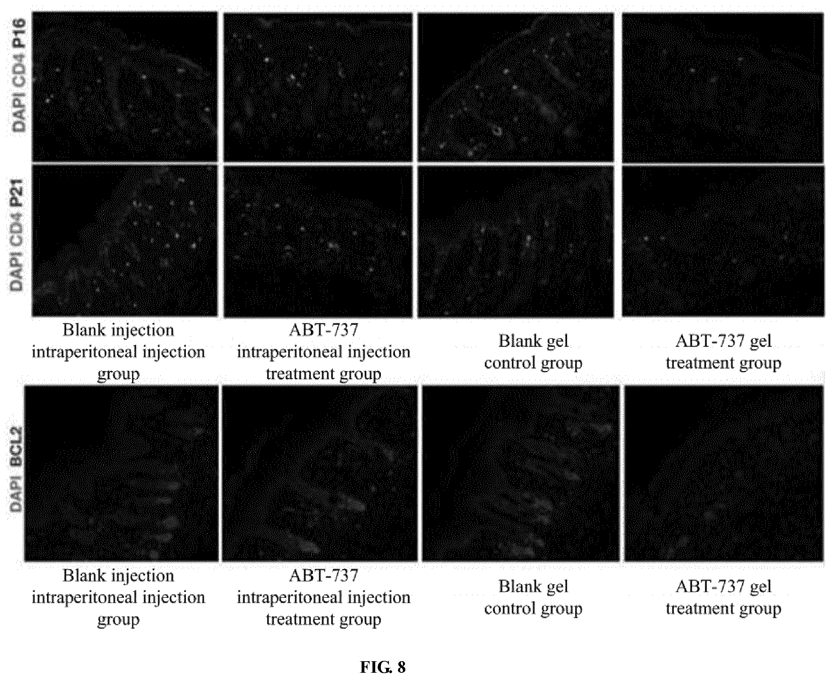

FIG. 8 is an immunohistochemical staining map of aging-related proteins P16 and P21, CD4 molecules and BCL-2 in the skin lesion sections of the mouse back modeling on the 7th day in the blank injection for subcutaneous injection group, the ABT-737 subcutaneous injection group, the blank gel control group and the ABT-737 gel treatment group provided in Example 6 of the present disclosure;

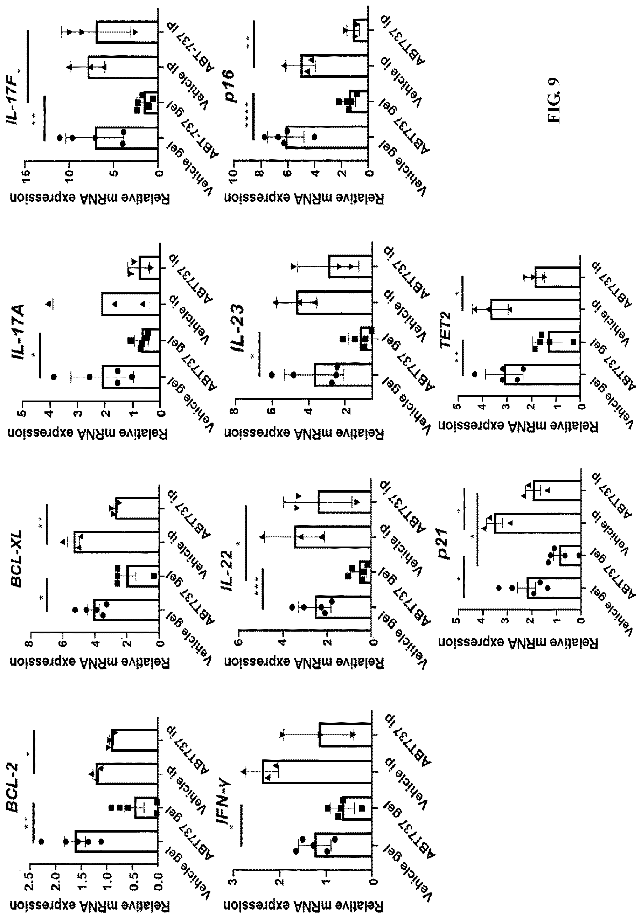

FIG. 9 is a graph showing mRNA expression levels of aging-related molecules P16 and P21, BCL-2 and various inflammatory factors taken from the skin of the mouse back modeling on the 7th day in the blank injection for subcutaneous injection group, the ABT-737 subcutaneous injection group, the blank gel control group and the ABT-737 gel treatment group provided in Example 6 of the present disclosure;

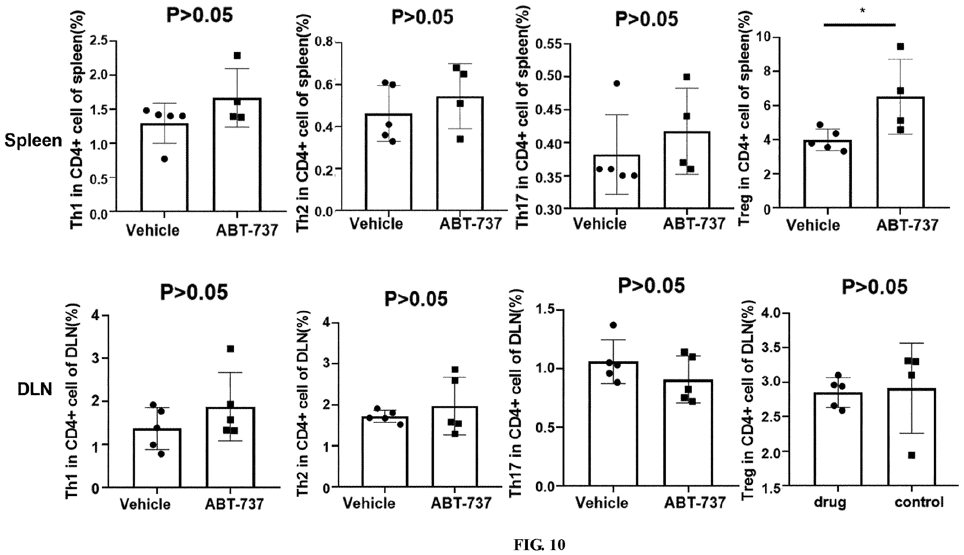

FIG. 10 is a diagram showing proportion changes of Th1, Th2, Th17 and Treg cells in mouse spleen and DLNs on the 7th day in the blank gel control group and the ABT-737 gel treatment group provided in Example 6 of the present disclosure by flow cytometry;

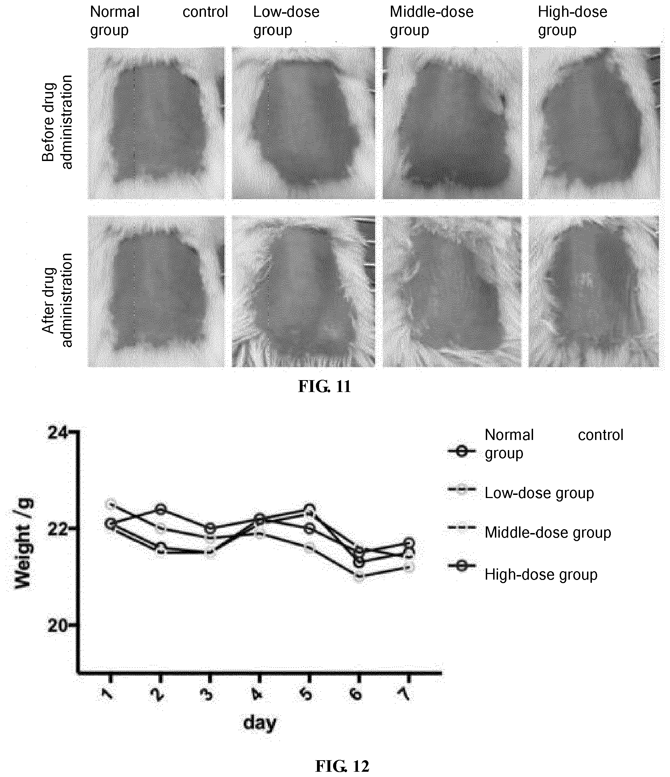

FIG. 11 is a diagram of skin lesions on a mouse back modeling on the 7th day in a normal control group, a low-dose group, a middle-dose group and a high-dose group provided in Example 7 of the present disclosure; and
FIG. 12 is a weight change chart during the test in the normal control group, the low-dose group, the middle-dose group and the high-dose group provided in Example 7 of the present disclosure.

### DETAILED DESCRIPTION OF THE EMBODIMENTS

The preferred embodiments of the present disclosure are described below with reference to the accompanying drawings. It should be understood that the preferred embodiments described herein are only used to illustrate the present disclosure, rather than to limit the present disclosure. If specific techniques or conditions are not indicated in the examples, the procedures shall be conducted in accordance with the techniques or conditions described in the literature in the art or in accordance with the product specification. Reagents or instruments not specified with manufacturers are all conventional products that can be purchased through proper channels.

Unless otherwise specified, technical and scientific terms used all have the same meanings as those as generally understood by a person of ordinary skill in the art to which the present disclosure belongs. All public references and the materials cited here will be incorporated by reference.

The present disclosure is further described in detail below with reference to the accompanying drawings and specific examples. It should be noted that the examples below are merely specific implementations of the present disclosure, and are used to describe rather than limiting the technical solutions of the present disclosure. Within the technical scope disclosed by the present disclosure, the addition or deletion of auxiliary medicinal ingredients or the changes of dosage form, the changes or deletion of proportion of each component, the addition of excipients to the pharmaceutical composition, or the equivalent replacements or changes according to the technical solution of the present disclosure and its inventive concept, shall be all covered within the protection scope of the present disclosure.

### Example 1

A 2.5 mg/ml BCL-2 inhibitor injection was provided.

Components were:
a 0.25% BCL-2 inhibitor (ABT-737) in mass percentage content, 10% DMSO, 40% PEG-300, 5% Tween-80, and PBS used as a balance.

Preparation method was:
each component was accurately weighed or accurately measured with a pipette according to the prescription; 120 µl of the DMSO was added with a pipette to 3 mg of the BCL-2 inhibitor (ABT-737) powder, and gently blown until the ABT-737 was completely dissolved to obtain a yellow clear solution; 480 µl of the PEG300, 60 µl of the Tween-80, and 537 µl of the PBS were added sequentially to the yellow clear solution, and the solution was mixed evenly by a vortexer to obtain the desired yellow clear solution.

### Example 2

A BCL-2 inhibitor gel was provided.

Components were:
a 0.05% BCL-2 inhibitor (ABT-737) in mass percentage content, 1% DMSO, 0.5% carbomer, and purified water as a balance.

Preparation method was:
each component was accurately weighed or accurately measured with a pipette according to the prescription; 0.5 g of the carbomer was dissolved in 98.5 g of the purified water completely by stirring to obtain a translucent solution, the translucent solution was wrapped with a plastic wrap, and swelled overnight in a refrigerator at 4°C to obtain a substrate; on a next day, under stirring, triethanolamine was slowly added to make the substrate to pH=7, to obtain a transparent gel substrate for later use; 50 mg of the ABT-737 powder was dissolved in 1 mL of the DMSO fully by gently blowing with a pipette; an ABT-737 solution was transferred to a spare gel substrate, and mixed evenly by stirring thoroughly to obtain a light yellow gel.

### Example 3

A 0 mg/ml BCL-2 inhibitor injection (blank injection) was provided.

Components were:
a 10% DMSO in mass percentage content, 40% PEG-300, 5% Tween-80, and PBS as a balance.

Preparation method was:
each component was accurately weighed or accurately measured with a pipette according to the prescription; 480 µl of the PEG300 and 60 µl of the Tween-80 were added to 120 µl of the DMSO sequentially to obtain a solution, and 540 µl of the PBS was added to the solution, and the solution was mixed evenly by a vortexer to obtain a desired transparent and clear solution.

### Example 4

a 0% BCL-2 inhibitor gel (blank gel) was provided.

Components were:
1% DMSO in mass percentage content, 0.5% carbomer, and purified water as a balance.

Preparation method was: each component was accurately weighed or accurately measured with a pipette according to the prescription; 0.5 g of the carbomer was dissolved in 98.5 g of the purified water completely by stirring to obtain a translucent solution, the translucent solution was wrapped with a plastic wrap, and swelled overnight in a refrigerator at 4°C to obtain a substrate; on a next day, under stirring, triethanolamine was slowly added to make the substrate to pH=7, to obtain a transparent gel substrate for later use; 1 mL of the DMSO was transferred to a spare gel substrate, and mixed evenly by stirring thoroughly to obtain a transparent substrate gel.

### Example 5

Effects of subcutaneous injection of 2.5 mg/ml BCL-2 inhibitor (ABT-737 injection) on imiquimod-induced psoriasis mouse model were studied.

16 healthy female Balb/c mice at the age of 8 weeks with a body weight of 20±2 g were taken, and depilated by 2×2 cm² on the back of the torso. After 24 h of depilation, it was confirmed that there was no abnormal change in depilated areas, and the mice were randomly divided into 4 groups, including: (1) a normal control group (3 mice): no special operations were conducted; (2) a psoriasis mouse model group (3 mice): psoriasis model was induced by external application of imiquimod; (3) a group of subcutaneous injection of ABT-737 injection (Example 1) to treat psoriasis mouse model (hereinafter referred to as ABT-737 subcutaneous injection group, 5 mice): psoriasis model was induced by external application of imiquimod, and subcutaneous injection of 25 mg/kg/day ABT-737 was conducted for treatment (that is, 200 µl of 2.5mg/ml ABT-737 injection was given to the above-mentioned mice); (4) a group of subcutaneous injection of blank injection (Example 3) to treat psoriasis mouse model (hereinafter referred to as blank injection for subcutaneous injection group, 5 mice): psoriasis model was induced by external application of imiquimod, and subcutaneous injection of blank injection was conducted in an equal volume with the ABT-737 subcutaneous injection group.

Except for the normal control group, the above-mentioned mice are all prepared with imiquimod-induced psoriasis models: 62.5 mg of 5% imiquimod (Sichuan Med Shine Pharmaceutical Co., Ltd.) was applied evenly with a cotton swab on the depilated areas on the back of the torso of the mouse, by once a day for 7 consecutive days with an interval of 24 h. After the modeling, the skin of the mouse back modeling in the psoriasis model group was taken for tissue paraffin embedding and routine section for HE staining observation. Compared with the normal control group, the model is established based on histological changes such as parakeratosis and spinous layer hypertrophy.

The following methodological tests were conducted during and after the modeling.
(1) From the day when the modeling was conducted to the day when the subcutaneous injection treatment was completed, skin appearance changes at the back modeling sites of the torso (at the depilated site in the normal control group) of 13 mice in the psoriasis mouse model group, the ABT-737 subcutaneous injection group, and the blank injection for subcutaneous injection group were daily observed (FIG. 1).
(2) The mice were sacrificed 24 h after the last subcutaneous injection treatment, and skin specimens on the back modeling sites of the torso of a total of 13 mice in the psoriasis mouse model group, the ABT-737 subcutaneous injection group, and the blank injection for subcutaneous injection group were taken, fixed with a 10% formaldehyde solution, and subjected to paraffin embedding, section and HE staining, the changes in the stratum corneum, granular layer, spinous cell layer, basal cell layer and dermis layer were observed in turn through an optical microscope, and the differences between each group was compared (FIG. 2). Immunohistochemical staining of p16, p21 and CD4 was conducted at the same time. The ratio of p16 and p21 positive cells of skin aging-related molecules between each group was compared under a multispectral microscope, and the infiltration degree of CD4⁺ T cells in the skin between each group was compared (FIG. 3).
(3) In addition, the skin specimens of the back modeling sites of the torso of a total of 10 mice in the ABT-737 subcutaneous injection group and the blank injection for subcutaneous injection group were taken, added to an RNA protection solution, cut and ground to prepare a single cell suspension. 1 mL of trizol was added to the single cell suspension and mixed thoroughly, RNA was extracted and reverse-transcribed into cDNA, and mRNA expression levels of aging-related molecules p16, p21, BCL-2 and corresponding genes of inflammatory factors were detected using real-time quantitative PCR technology (FIG. 4).
(4) After the mice were sacrificed, the spleens and bilateral DLNs (inguinal and axillary lymph nodes) of 10 mice in the ABT-737 subcutaneous injection group and the blank injection for subcutaneous injection group were taken out immediately, ground, filtered and washed with PBS, and centrifuged to prepare a single cell suspension; 2×10⁶ cells were taken from each sample, the cell membrane surface molecules CD4 and CD25 were stained, and dead and living cells were distinguished using Zombie NIR TM Fixable Viability Kit, and nuclear membrane was ruptured using a nuclear membrane rupture agent, nucleus Foxp3 transcription factor was stained, and flow cytometry was conducted (FIG. 5).
(5) 2.5×10⁶ cells were taken from the single cell suspension of the spleen and DLNs of the above 10 mice, a complete medium (a 1640 solution containing 10% FBS) was added, 2 µl of an ionomycin-Golgi blocker was added to each sample; the cells were transferred to each well of a 24-well fine culture plate, and the complete medium was added to ensure that a total volume in the well is 1 ml; the cell culture plate was placed in a 37°C cell incubator, and stained cell membrane surface molecules CD4 were harvested after culturing for 5 h; dead and living cells were distinguished using Zombie NIR TM Fixable Viability Kit, the cell membrane was ruptured using the cell membrane rupture agent, and cell endocrine factors IFN-γ, IL-4 and IL-17A were stained using flow cytometry antibodies, and flow cytometry was conducted after staining (FIG. 5).

The results were as follows:
(1) In terms of the appearance of the skin tissues at the modeling site, the psoriasis model shows significant skin swelling, skin lesions darkening, epidermis thickening, skin grooves deepening and skin ridges raising; the skin lesions of the mice in the blank injection for subcutaneous injection group are slightly ameliorated compared with the psoriasis model group, but still have significant symptoms mentioned above; compared with the blank injection subcutaneous for injection group and the psoriasis model group, the mice in the ABT-737 subcutaneous injection group have significant improvement in skin swelling, skin thickening and scales (FIG. 1).
(2) The pathological sections of the skin on the back of the torso of each group of mice are observed under an optical microscope: in the psoriasis mouse model group, the stratum corneum is broken, the granular layer is thinned or disappears, the parakeratosis is obvious, the spinous layer is thickened, and the basal cell proliferation is active, dermal inflammatory cells are more infiltrated with dermal fibrosis, showing a pathological state of the psoriasis; in the ABT-737 subcutaneous injection group, the downward extension of epidermal protrusions on the back skin lesions of the mice is significantly reduced, the spinous cell layer is thinned, and the dermal inflammatory cells are significantly reduced; in the blank injection for subcutaneous injection group, the epidermal protrusions on the back skin lesions of the mouse are obviously extended downward and widened, the spinous layer became thicker, and the dermal inflammatory cells are reduced, but the effects above are not as good as that of the ABT-737 subcutaneous injection group (FIG. 2).
(3) Immunohistochemical staining is conducted on the p16, p21 and CD4 in the skin pathological sections of the back modeling site of the torso of the mouse and observation is conducted by a multispectral microscope: compared with the blank injection for subcutaneous injection group, in the ABT-737 subcutaneous injection group, the proportion of senescent cells staining positive in p16 and p21 in the paraffin sections of the skin lesions on the back of mice is significantly reduced; the proportion of CD4⁺ cells infiltrated by dermal inflammation is significantly reduced (FIG. 3).
(4) Real-time quantitative PCR detection of the mRNA expression levels of aging-related molecules P16, P21, BCL-2 and the corresponding genes of various inflammatory factors in the skin of the back modeling sites of the mouse torso: compared with the blank injection for subcutaneous injection group, in the ABT-737 subcutaneous injection group, the mRNA expression levels of genes corresponding to P16, P21, BCL-2, INF-γ and IL17A decrease significantly (FIG. 4).
(5) Flow cytometric detection of expression levels in Th subgroups and Treg cells in mouse spleen and DLNs: compared with the blank injection for subcutaneous injection group, in the ABT-737 subcutaneous injection group, there is no statistical difference in the proportion of Th1, Th2, Th17 and Treg cells in the spleen and DLNs accounting for the CD4⁺ T cells (FIG. 5).

The results show that: compared with the blank injection for subcutaneous injection group, the ABT-737 subcutaneous injection group can significantly reduce or remove the psoriasis symptoms of psoriasis model mice induced by imiquimod, such as skin swelling, skin thickening, and covering scales; in addition, by reducing epidermal protrusion extension and dermal inflammatory cell infiltration, the hypertrophic spinous layer was thinned to thin the epidermis, inhibiting hyperplasia and abnormal keratinization of the epidermis, such that the skin lesions tend to subside to significantly improve the pathological state of psoriasis. In addition, the proportion of senescent cells staining positive in p16 and p21 of the paraffin sections of skin lesions on the back of mice in the ABT-737 subcutaneous injection group is significantly reduced compared with that in the psoriasis model group and the blank injection for subcutaneous injection group; the proportion of inflammatory infiltration CD4⁺ cells is significantly reduced. Meanwhile, the mRNA expression levels of corresponding genes of the P16, P21, BCL-2, INF-γ and IL17A in the skin of the modeling site of the torso back of the mice in the ABT-737 subcutaneous injection group decrease significantly. In addition, compared with the blank injection for subcutaneous injection group, in the ABT-737 subcutaneous injection group, there is no statistical difference in the proportion of Th1, Th2, Th17 and Treg cells in the spleen and DLNs accounting for the CD4⁺ T cells. It shows that the BCL-2 inhibitor (ABT-737 injection) can be used as a drug for treating the psoriasis, and only relieves the psoriasis skin lesions at the skin level, without any systemic damage.

### Example 6

Effects of external application of BCL-2 inhibitor ABT-737 gel (Example 2) and intraperitoneal injection of 2.5 mg/ml BCL-2 inhibitor ABT-737 injection (Example 1) on imiquimod-induced psoriasis mouse model was studied.

19 healthy female Balb/c mice at the age of 8 weeks with a body weight of 20±2 g were taken, and depilated by 2x2 cm² on the back of the torso. After 24 h of depilation, it was confirmed that there was no abnormal change in depilated areas, and the mice were randomly divided into 5 groups, including: (1) a psoriasis mouse model group (3 mice): psoriasis model was induced by external application of imiquimod; (2) a group external application of ABT-737 gel (Example 2) for treating psoriasis mouse model (hereinafter referred to as ABT-737 gel treatment group, 5 mice): psoriasis model was induced by external application of imiquimod, and each mouse was treated with ABT-737 gel 0.5 g/day for external application; (3) a group of external application of blank gel (Example 4) to treat the psoriasis mouse model (hereinafter referred to as the blank gel control group, 5 mice): the psoriasis model was induced by external application of imiquimod, and each mouse was treated with blank gel the same amount as the ABT-737 treatment group by external application; (4) a group of intraperitoneal injection of BCL-2 inhibitor ABT-737 injection (Example 1; hereinafter referred to as ABT-737 intraperitoneal injection treatment group, 3 mice): psoriasis model was induced by external application of imiquimod, and each mouse was treated by intraperitoneal injection of 25 mg/kg/day of ABT-737 (that is, 200 µl of 2.5 mg/ml ABT-737 injection was given to the above-mentioned mice); (5) a group of blank injection for intraperitoneal injection (Example 3; hereinafter referred to as blank injection for intraperitoneal injection group, 3 mice): psoriasis model was induced by external application of imiquimod, and each mouse was treated by intraperitoneal injection of 200 µl of blank injection.

The above-mentioned mice were prepared with imiquimod-induced psoriasis models, and the specific steps were the same as in Example 5.

In the ABT-737 gel treatment group and the blank gel control group, during the modeling of imiquimod-induced psoriasis mouse model, it is also necessary to conduct external application of the ABT-737 gel or the blank gel on the back modeling skin of the mouse for treatment. After depilation for 18 h, the ABT-737 gel treatment group and the blank gel control group were treated with 0.5 g/piece of ABT-737 gel or blank gel by external application, where a frequency was once a day for 6 consecutive days, with an interval of 24 h each time. 6 h after the end of the gel treatment by external application every day, the psoriasis mouse model was induced by external application of imiquimod.

In the ABT-737 intraperitoneal injection treatment group and the blank injection for intraperitoneal injection group, during the modeling of imiquimod-induced psoriasis mouse model, it is also necessary to conduct treatment on the mouse abdomen by ABT-737 intraperitoneal injection or blank injection for intraperitoneal injection. When the mice in the ABT-737 gel treatment group and the blank gel control group were treated, the mice in the ABT-737 intraperitoneal injection treatment group and the blank injection for intraperitoneal injection group were intraperitoneally injected with 200 µl of 2.5 mg/ml ABT-737 injection or an equal volume of blank injection for treatment.

The following methodological tests were conducted during and after the modeling.
(1) From the day when the modeling was conducted to the day when the application was completed, skin appearance changes at the back modeling sites of the torso of 19 mice were daily observed (FIG. 6).
(2) The mice were sacrificed 24 h after the last gel treatment by external application, and skin specimens on the back modeling site of the torso of a total of 19 mice were taken, fixed with 10% formaldehyde solution, and subjected to paraffin embedding, section and HE staining, the changes in the stratum corneum, granular layer, spinous cell layer, basal cell layer and dermis layer were observed in turn through an optical microscope, and the differences between each group was compared (FIG. 7). Meanwhile, in the ABT-737 gel treatment group, the blank gel control group, the ABT-737 intraperitoneal injection treatment group, and the blank injection for intraperitoneal injection group, the paraffin sections of the skin lesions on the back modeling sites of the torso of the mouse were subjected to immunohistochemical staining on p16 , P21, CD4 and BCL2; under a multispectral microscope, the changes in the proportion of senescent cells positive in skin aging-related molecules p16 and p21, the degree of skin CD4⁺ T cell infiltration and the changes in the proportion of BCL2 positive cells between the groups were compared (FIG. 8).
(3) In addition, the skin specimens of the back modeling sites of the torso of the mice in the ABT-737 gel treatment group, the blank gel control group, the ABT-737 intraperitoneal injection treatment group and the blank injection for intraperitoneal injection group were taken, added to an RNA protection solution, cut and ground to prepare a single cell suspension. 1 mL of trizol was added to the single cell suspension and mixed thoroughly, RNA was extracted and reverse-transcribed into cDNA, and mRNA expression levels of aging-related molecules p16, p21, Tet2, BCL-2, BCL-xL and corresponding genes of inflammatory factors were detected using the real-time quantitative PCR technology (FIG. 9).
(4) After the mice were sacrificed, the spleens and bilateral DLNs (inguinal and axillary lymph nodes) mice in the ABT-737 gel treatment group and blank gel control group were taken out immediately, ground, filtered and washed with PBS, and centrifuged to prepare a single cell suspension; 2×10⁶ cells were taken from each sample, the cell membrane surface molecules CD4 and CD25 were stained, and dead and living cells were distinguished using Zombie NIR TM Fixable Viability Kit, and nuclear membrane was ruptured using a nuclear membrane rupture agent, nucleus Foxp3 transcription factor was stained, and flow cytometry was conducted (FIG. 10).
(5) 2.5×10⁶ cells were taken from the single cell suspension of the spleen and DLNs of the above mice in the ABT-737 gel treatment group and the blank gel control group, a complete medium (a 1640 solution containing 10% FBS) was added, 2 µl of an ionomycin-Golgi blocker was added to each sample; the cells were transferred to each well of a 24-well fine culture plate, and the complete medium was added to ensure that a total volume in the well is 1 ml; the cell culture plate was placed in a 37°C cell incubator, and stained cell membrane surface molecules CD4 were harvested after culturing for 5 h; dead and living cells were distinguished using Zombie NIR TM Fixable Viability Kit, the cell membrane was ruptured using the cell membrane rupture agent, and cell endocrine factors IFN-γ, IL-4 and IL-17A were stained using flow cytometry antibodies, and flow cytometry was conducted after staining (FIG. 10).

The results were as follows:
(1) In terms of the appearance of the skin tissues at the modeling site, the psoriasis model shows significant skin swelling, skin lesions darkening, epidermis thickening, skin grooves deepening and skin ridges raising; the skin lesions of the mice in the blank gel control group are slightly ameliorated compared with the psoriasis model group, but still have significant symptoms mentioned above; compared with the blank gel control group and the psoriasis model group, the mice in the ABT-737 gel treatment group have significant improvement in skin swelling, skin thickening and scales. For the improvement of the skin lesions of the mouse model, the blank injection for intraperitoneal injection group is the same as the blank gel control group; the ABT-737 intraperitoneal injection treatment group is better than the psoriasis model group, the blank gel control group and the blank injection for intraperitoneal injection group, but is slightly inferior to the ABT-737 gel treatment group (FIG. 6).
(2) The pathological sections of the skin on the back of the torso of each group of mice are observed under an optical microscope: in the psoriasis mouse model group, the stratum corneum is broken, the granular layer is thinned or disappears, the parakeratosis is obvious, the spinous layer is thickened, and the basal cell proliferation is active, dermal inflammatory cells are more infiltrated with dermal fibrosis, showing a pathological state of the psoriasis; in the ABT-737 gel treatment group, the downward extension of epidermal protrusions on the back skin lesions of the mice is significantly reduced, the spinous cell layer is thinned, and the dermal inflammatory cells are significantly reduced; in the blank gel control group, the epidermal protrusions on the back skin lesions of the mouse are obviously extended downward and widened, the spinous layer became thicker, and the dermal inflammatory cells are reduced, but the effects above are not as good as that of the ABT-737 gel treatment group; for the improvement of the skin lesions of the mouse model, the blank injection for intraperitoneal injection group is the same as the blank gel control group; the ABT-737 intraperitoneal injection treatment group is better than the psoriasis model group, the blank gel control group and the blank injection for intraperitoneal injection group, but is slightly inferior to the ABT-737 gel treatment group (FIG. 7).
(3) Immunohistochemical staining is conducted on the p16, p21, CD4 and BCL-2 in the skin pathological sections of the modeling site on the back of the torso of the mouse and observation is conducted by a multispectral microscope: compared with the blank gel control group, in the ABT-737 gel treatment group, the proportion of senescent cells staining positive in p16 and p21 on the back skin pathological section of the mice is significantly reduced; the proportion of CD4⁺ cells infiltrated by dermal inflammation is significantly reduced ; the epidermal BCL2 positive cells are significantly reduced; in the blank injection for intraperitoneal injection group, the proportion of cells staining positive in p16, p21, CD4 and BCL2 in the skin pathological sections of the mouse modeling sites is basically the same as that of the blank gel control group; for the reduction of p16, p21, CD4 and BCL2 positive cells, the ABT-737 intraperitoneal injection treatment group is better than the blank gel control group and the blank injection for intraperitoneal injection group, but is slightly inferior to the ABT-737 gel treatment group (FIG. 8).
(4) Real-time quantitative PCR detection of the mRNA expression levels of aging-related molecules P16, P21, Tet2 and BCL-2 and the corresponding genes of various inflammatory factors in the skin of the back modeling sites of the mouse torso: compared with the psoriasis model group, the blank gel control group, the blank injection for intraperitoneal injection group and the ABT-737 intraperitoneal injection treatment group, the mRNA expression levels of the corresponding genes of P16, P21, BCL-2 and IL-22, IL-23 and Tet2 in the ABT-737 gel treatment group are significantly reduced (FIG. 9).
(5) Flow cytometric detection of expression levels in Th subgroups and Treg cells in mouse spleen and DLNs in the ABT-737 gel treatment group and the blank gel control group: compared with the blank gel control group, in the ABT-737 gel treatment group, there is no statistical difference in the proportion of Th1, Th2, Th17 cells in the spleen and DLNs accounting for the CD4⁺ T cells; while the proportion of spleen Treg cells in CD4⁺ T cells increases, which is statistically different (FIG. 10).

The results show that: compared with the blank gel control group, the blank injection for intraperitoneal injection group and the ABT-737 intraperitoneal injection treatment group, the BCL-2 inhibitor ABT-737 gel can significantly reduce or remove the psoriasis symptoms of psoriasis model mice induced by imiquimod, such as skin swelling, skin thickening, and covering scales; in addition, by reducing epidermal protrusion extension and dermal inflammatory cell infiltration, the hypertrophic spinous layer was thinned to thin the epidermis, inhibiting hyperplasia and abnormal keratinization of the epidermis, such that the skin lesions tend to subside to significantly improve the pathological state of psoriasis; compared with the psoriasis model group, the blank gel control group, the blank injection for intraperitoneal injection group and the ABT-737 intraperitoneal injection treatment group, in the ABT-737 gel treatment group, the senescent cells staining positive in p16 and p21 in the skin pathological sections of the mouse back are significantly reduced; the proportion of inflammatory infiltration CD4⁺ cells is significantly reduced, and the epidermal BCL2 positive cells are significantly reduced. Meanwhile, in the ABT-737 gel treatment group, the mRNA expression levels of corresponding genes of the P16, P21, BCL-2, IL-22 and IL-23 in the skin decrease significantly, and there is statistically significant differences that the ABT-737 gel treatment group reduces the mRNA expression levels of the genes of IL-17F, IL-22 and p21 compared with the ABT-737 intraperitoneal injection treatment group; the proportion of spleen Treg cells in CD4⁺ T cells increases; but compared with the blank gel control group, in the ABT-737 gel treatment group, there is no significant difference in the proportion of Th1, Th2 and Th17 in the spleen and DLNs accounting for the CD4⁺ T cells. This indicates that the BCL-2 inhibitor (ABT-737) gel can be used as a drug for treating psoriasis, with little systemic impact and mostly relieving the psoriasis skin lesions at the skin level.

### Example 7

External application of BCL-2 inhibitor ABT-737 gel (the substrate was the same as in Example 2, except that the content of ABT-737 was different) was conducted for acute toxicity test on intact skin of the mouse.

12 female Balb/c mice at the age of 8 weeks were taken, and depilated by 2×2 cm² on the back of the torso. After 24 h of depilation, it was confirmed that there was no abnormal change in depilated areas. The Balb/c mice were randomly divided into four groups, including: (1) a normal control group: there were 3 mice in total, no other treatments were conducted; (2) 0.05% ABT-737 gel for external application (hereinafter referred to as a low-dose group, 3 mice): 0.05% ABT-737 gel was used for external application, with a dose of 0.5 g/piece/day, for 7 consecutive days, at an interval of 24 h; (3) 0.1% ABT-737 gel for external application (hereinafter referred to as a middle-dose group, 3 mice): 0.1% ABT-737 gel was used for external application, with a dose of 0.5 g/piece/day, for 7 consecutive days, at an interval of 24 h; (4) 0.2% ABT-737 gel for external application (hereinafter referred to as a high-dose group, 3 mice): 0.2% ABT-737 gel was used for external application, with a dose of 0.5 g/piece/day, for 7 consecutive days, at an interval of 24 h.

During the experiment, the following methodological monitoring was conducted: daily observation was conducted to confirm the reaction of the back skin (FIG. 11), and the animal body weight (FIG. 12) of each group of mice after the gel was completely absorbed; and the animal reaction and death number were observed and recorded for 7 consecutive days, dead animals were timely autopsied, and histomorphological microscopy was conducted for those with abnormalities.

The results show that after drug application, the back skin of the animals has no obvious irritation (FIG. 11), and there is no difference in body weight (FIG. 12). The animals in each group are generally in desirable conditions, have no obvious changes in activities and behaviors, and have normal diet and feces. None of the animals in the four groups died.

Although the present disclosure has been described in detail through the above examples, the examples are only a part rather than all of the examples of the present disclosure. All other examples obtained by persons based on these examples without creative efforts shall fall within a protection scope of the present disclosure.

## Claims

1. Use of a B-cell lymphoma 2 (BCL-2) inhibitor in preparation of a drug for treating aging-related skin diseases.

2. The use according to claim 1, wherein the BCL-2 inhibitor can inhibit members of a BCL-2 anti-apoptotic protein family, comprising at least one selected from the group consisting of BCL-2, BCL-xL and BCL-w.

3. The use according to claim 1 or 2, wherein the BCL-2 inhibitor is at least one selected from the group consisting of ABT-737 and ABT-263.

4. The use according to claim 3, wherein the BCL-2 inhibitor is ABT-737.

5. The use according to claim 1, wherein the drug is any pharmaceutically-acceptable pharmaceutical preparation prepared by combining a pharmaceutically-effective amount of the BCL-2 inhibitor with pharmaceutically-acceptable accessories.

6. The use according to claim 5, wherein the drug is any pharmaceutical preparation prepared by the BCL-2 inhibitor for external, topical, transdermal, intradermal, systemic, inhalation, intratracheal or intubation administrations.

7. The use according to claim 5 or 6, wherein the pharmaceutical preparation is at least one selected from the group consisting of an injection preparation and an external preparation.

8. The use according to claim 7, wherein the injection preparation is a subcutaneous injection preparation.

9. The use according to claim 7, wherein the external preparation is at least one selected from the group consisting of gels, emulsifiable pastes, emulsions, creams, ointments, pastes, lotions, patches, dressings, coatings, cataplasms, aerosols and sprays.

10. The use according to claim 1, wherein the aging-related skin diseases are at least one skin disease or condition selected from the group consisting of: eczema, psoriasis, hyperpigmentation, mole, rash, atopic dermatitis, urticaria, diseases and disorders related to photosensitivity or photoaging and wrinkles, itching, dullness, eczema outbreak, eosinophilic skin disease, reactive neutrophil skin disease, pemphigus; pemphigoid, immune bullous skin disease, skin fibrous tissue cell proliferation, keloids, skin lymphoma and cutaneous lupus.

11. The use according to claim 1 or 10, wherein the aging-related skin disease is psoriasis.

12. The use according to claim 11, wherein the psoriasis is at least one selected from the group consisting of psoriasis vulgaris, erythroderma psoriasis, psoriatic arthritis and pustular psoriasis.

13. Use of a BCL-2 inhibitor in preparation of a drug for aging-related skin diseases in progressive, stationary or regressive phases.

14. The use according to claim 1 or 13, wherein the BCL-2 inhibitor is combined with other drugs for treating the aging-related skin diseases to prepare a composite drug.

15. A drug for treating aging-related skin diseases, comprising a pharmaceutically-effective amount of a BCL-2 inhibitor.

16. The drug for treating aging-related skin diseases according to claim 15, wherein the drug has a dosage form of an injection preparation or an external preparation.

17. The drug for treating aging-related skin diseases according to claim 16, wherein the injection preparation comprises the BCL-2 inhibitor, a solvent A, a solvent B, a solvent C and a surfactant;
the solvent A is an organic solvent; the solvent B is an alcohol solvent; and the solvent C is water or a buffer.

18. The drug for treating aging-related skin diseases according to claim 17, wherein the solvent A is at least one selected from the group consisting of dimethyl sulfoxide (DMSO), dimethyl formamide (DMF), dimethyl acetamide (DMAC or DMA), N-methylpyrrolidone (NMP) and DMAF;
the solvent B is at least one selected from the group consisting of PEG-300, PEG-400, polyethylene glycol or propylene glycol, ethanol and glycerin;
the solvent C is at least one selected from the group consisting of a phosphate-buffered saline (PBS), a carbonate buffer, an acetate buffer, a borate buffer, a citrate buffer, a citrate buffer and purified water; and
the surfactant is at least one selected from the group consisting of polysorbate, Tween-80, Tween-20, polyoxy castor oil, nonionic surfactant HS15, vitamin E polyethylene glycol succinate, Pluronic F68 and gelucire 44/14.

19. The drug for treating aging-related skin diseases according to claim 17, wherein in the injection preparation, the BCL-2 inhibitor has a mass percentage of 0.001-5%;
the solvent A has a mass percentage of 0.1-15%;
the solvent B has a mass percentage of 20-60%;
the surfactant has a mass percentage of 1-10%; and
the solvent C is used as a balance.

20. The drug for treating aging-related skin diseases according to claim 19, wherein in the injection preparation, the BCL-2 inhibitor has a mass percentage of 0.05-1%; and the solvent A has a mass percentage of 5-10%.

21. The drug for treating aging-related skin diseases according to claim 16, wherein optionally, the external preparation further comprises at least one of a penetration enhancer and a preservative.

22. The drug for treating aging-related skin diseases according to claim 21, wherein the penetration enhancer is one or more selected from the group consisting of menthol, borneol, azone, oleic acid, and *Cnidium monnieri* volatile oil; and the penetration enhancer has a weight percentage of not more than 10%.

23. The drug for treating aging-related skin diseases according to claim 21, wherein the preservative is at least one selected from the group consisting of ethyl paraben, ethyl paraben, sodium benzoate, benzalkonium bromide, chlorobutanol and sorbic acid; and the preservative has a weight percentage of not more than 10%.

24. The drug for treating aging-related skin diseases according to claim 16, wherein
preparation raw materials of the external preparation comprise the BCL-2 inhibitor, a solvent D, a substrate and a solvent E;
the solvent D is one or more selected from the group consisting of DMSO, DMF, DMAC or DMA, NMP and DMAF;
the substrate is one or more selected from the group consisting of hydroxypropyl methylcellulose, sodium carboxymethyl cellulose, carbomer, hyaluronic acid, polyvinyl alcohol, sodium alginate, sodium hyaluronate, methylcellulose, chitosan and chitin; and
the solvent E is one or more selected from the group consisting of a PBS, a carbonate buffer, an acetate buffer, a borate buffer, a citrate buffer, a citrate buffer and purified water.

25. The drug for treating aging-related skin diseases according to claim 24, wherein in the external preparation, the BCL-2 inhibitor has a mass percentage of 0.001-5%;
the solvent D has a mass percentage of 0.1-15%;
the substrate has a mass percentage of 0.01-10%; and
the solvent E is used as a balance.

26. The drug for treating aging-related skin diseases according to claim 25, wherein in the external preparation, the BCL-2 inhibitor has a mass percentage of 0.02-1%; the solvent D has a mass percentage of 0.5-1.5%; and the substrate has a mass percentage of 0.1-1.0%.

27. The drug for treating aging-related skin diseases according to claim 16, wherein optionally, the external preparation further comprises at least one of a humectant, a solvent F, a penetration enhancer and a preservative; and
the solvent F is one or more selected from the group consisting of PEG-300, PEG-400, polyethylene glycol, propylene glycol, ethanol and glycerin.

28. The drug for treating aging-related skin diseases according to claim 27, wherein the humectant is selected from the group consisting of propylene glycol and glycerin; the humectant has a weight percentage of not more than 10%; and
the solvent F has a weight percentage of not more than 10%.

29. The drug for treating aging-related skin diseases according to any one of claims 15-28, wherein the drug further comprises other active ingredients for treating the aging-related skin diseases.

30. A method for treating aging-related skin diseases using the drug for treating aging-related skin diseases according to any one of claims 15-28, comprising the following steps: allowing patients suffering from aging-related skin diseases to ingest the drug for treating aging-related skin diseases; and
a method of ingestion comprises injection and/or external application.
